# EUROPEAN PATENT SPECIFICATION

(11) **EP 4 440 540 B1**
(45) Date of publication and mention of the grant of the patent: **18.06.2025**
(21) Application number: 22814334.3
(22) Date of filing: 08.11.2022
(51) Int. Cl.: A61K 8/49, A61K 8/67, A61K 8/73, A61Q 19/02, A61Q 19/08

(54) **PERSONAL CARE COMPOSITION COMPRISING RETINOID, MODIFIED STARCH AND SORBITAN FATTY ESTER**
KÖRPERPFLEGEZUSAMMENSETZUNG MIT RETINOID, MODIFIZIERTER STÄRKE UND SORBITANFETTSÄUREESTER
COMPOSITION DE SOINS PERSONNELS COMPRENANT UN RÉTINOÏDE, DE L'AMIDON MODIFIÉ ET UN ESTER GRAS DE SORBITAN

(30) Priority: 01.12.2021 WO PCT/CN2021/134721; 13.01.2022 EP 22151278
(43) Date of publication of application: 09.10.2024
(73) Proprietor: Unilever IP Holdings B.V., 3013 AL Rotterdam (NL); Unilever Global IP Limited, Wirral, Merseyside CH62 4ZD (GB)
(72) Inventor: BIAN, Wei, 6708 WH Wageningen (NL); WEI, Ping, 6708 WH Wageningen (NL)
(74) Representative: James, Helen Sarah
(86) International application number: PCT/EP2022/081032
(87) International publication number: WO 2023/099130

(56) References cited:
- EP-A1- 1 743 624
- WO-A1-2008/055931
- WO-A1-2021/026565
- DATABASE GNPD [online] MINTEL; 8 April 2011 (2011-04-08), ANONYMOUS: "Anti-Wrinkle Gel Cream", XP055880560, retrieved from https://www.gnpd.com/sinatra/recordpage/1527416/ Database accession no. 1527416

## Description

### Field of the Invention

The present invention relates to a personal care composition. Particularly, the present invention relates to a personal care composition comprising retinoids having improved color stability.

### Background of the Invention

Skin care compositions containing retinoids, such as retinol, have become quite prominent in recent years. Retinoids are known to provide a wide spectrum of skin benefits such as skin brightening, wrinkle treatment, and etc.

Unfortunately, retinoids have problem of discoloration, especially in the presence of water. Personal care compositions, however, almost always include substantial amounts of water, in order to deliver an aesthetically acceptable appearance and tactile properties. Discoloration is of concern because consumers are sensitive to changes in color appearance of cosmetic compositions that they use, especially in cases where people may believe that color change cues degradation of chemical activeness. EP1743624, WO2021026565 and WO2008055931 describe compositions comprising retinoids, starch and sorbitan fatty esters.

Therefore, the present inventors have recognized a need to develop a personal care composition containing retinoids having improved color stability. It was surprisingly found that by including sorbitan fatty acid ester with weight ratio of the sorbitan fatty acid ester to the retinoid is 0.4:1 to 1.4:1 in the presence of modified starch, the discoloration problem of the personal care containing retinoids was considerably reduced.

### Summary of the Invention

In a first aspect, the present invention is directed to a personal care composition according to claim 1 comprising retinoid, 0.01 to 10% of modified starch by weight of the composition, and sorbitan fatty acid ester, wherein the weight ratio of the sorbitan fatty acid ester to the retinoid is 0.4:1 to 1.4:1 and the weight ratio of the total modified starches to the sorbitan fatty acid ester is 1:1 to 70:1.

In a second aspect, the present invention is directed to a method for providing skin brightening, and/or anti-wrinkle benefit to skin comprising the step of applying a composition of the present invention on the desired skin surface.

In a third aspect, the present invention is directed to use of a composition of the present invention for providing skin brightening, and/or anti-wrinkle benefit on the desired skin surface.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The composition of the present invention comprises a retinoid. Typically, the retinoid comprises retinyl ester, retinol, retinal, retinoic acid or a mixture thereof. More preferably the retinoid comprises retinol, retinyl ester, or a mixture thereof and even more preferably the retinoid is selected from retinol, retinyl ester, or a mixture thereof.

The term "retinol" includes the following isomers of retinol: all-trans-retinol, 13-cis-retinol, 11-cis-retinol, 9-cis-retinol, 3,4-didehydro-retinol, 3,4-didehydro-13-cis-retinol; 3,4-didehydro-11-cis-retinol; 3,4-didehydro-9-cis-retinol. Preferred isomers are selected from all-trans-retinol, 13-cis-retinol, 3,4-didehydro-retinol, and 9-cis-retinol. Most preferred retinol is all-trans-retinol, due to its wide commercial availability.

Retinyl ester is an ester of retinol. The term "retinol" has been defined above. Retinyl esters suitable for use in the present invention are preferably C₁-C₃₀ esters of retinol, more preferably C₂-C₂₀ esters of retinol, and most preferably C₂, C₃, and C₁₆ esters of retinol. Examples of retinyl esters comprises retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl myristate, retinyl pentadecanoate, retinyl heptadecanoate, retinyl stearate, retinyl isostearate, retinyl nonadecanoate, retinyl arachidonate, retinyl behenate, retinyl linoleate, retinyl oleate or a combination thereof. The retinyl ester for use in the present invention is preferably selected from retinyl palmitate, retinyl acetate, retinyl linoleate, retinyl oleate, retinyl propionate or a mixture thereof. More preferably the retinyl ester is selected from retinyl palmitate, retinyl acetate, retinyl propionate, or a mixture thereof. Most preferably the retinyl ester is selected from retinyl palmitate, retinyl propionate, or a mixture thereof.

Particularly preferred retinoid is selected from all-trans-retinol, retinyl palmitate, retinyl acetate, retinyl propionate, or a mixture thereof. Most preferably the retinoid is selected from retinyl palmitate, retinyl propionate, or a mixture thereof.

Preferably, retinoid is employed in the composition in an amount of 0.0001 to 5% by weight of the composition, more preferably in an amount of 0.005 to 3%, even more preferably from 0.05 to 1% and most preferably in an amount of 0.1 to 0.5% by weight of the composition.

The composition of the present invention comprises sorbitan fatty acid ester. Sorbitan fatty esters are a class of emulsifiers used in personal care composition. The sorbitan fatty acid ester is sorbitan C₈₋₂₄ fatty acid ester, more preferably sorbitan C₁₀₋₂₀ fatty acid ester. Preferably, the sorbitan fatty esters comprises polyoxyethylene sorbitan fatty acid esters and more preferably the sorbitan fatty ester is polyoxyethylene sorbitan fatty acid ester. Polyoxyethylene sorbitan fatty acid esters are polyethylene glycol (PEG)-ylated sorbitan (a derivative of sorbitol), esterified with fatty acids and are often called Polysorbate or tween.

Polyoxyethylene sorbitan fatty acid esters are preferably polyoxyethylene sorbitan C₈₋₂₄ fatty acid esters, more preferably polyoxyethylene sorbitan C₁₀₋₂₀ fatty acid esters. Preferably, the total number of oxyethylene group in the sorbitan fatty acid ester is 5 to 50, more preferably 10 to 30, and even more preferably 15 to 25.

Preferably, the sorbitan fatty acid esters comprises Polysorbate 20, Polysorbate 40, Polysorbate 60, Polysorbate 80, or a mixture thereof. More preferably, the sorbitan fatty acid esters comprises Polysorbate 20, Polysorbate 80, or a mixture thereof and most preferably the sorbitan fatty acid ester is Polysorbate 20.

Preferably, the sorbitan fatty acid ester is present in amount of 0.15 to 0.4%, more preferably 0.2 to 0.35% by weight of the composition. Preferably, the polyoxyethylene sorbitan fatty acid ester is present in amount of 0.15 to 0.4%, preferably 0.2 to 0.35% by weight of the composition. Preferably, the Polysorbate 20 is present in amount of 0.15 to 0.4%, preferably 0.2 to 0.35% by weight of the composition.

Preferably, the weight ratio of the sorbitan fatty acid ester to the retinoid is 0.55:1 to 1.2:1. Preferably, the weight ratio of the polyoxyethylene sorbitan fatty acid ester to the retinoid is 0.4:1 to 1.4:1, more preferably 0.55:1 to 1.2:1. Preferably, the weight ratio of the Polysorbate 20 to the retinoid is 0.4:1 to 1.4:1, more preferably 0.55:1 to 1.2:1.

The composition of the present invention comprises modified starch. Modified starch refers to starch having a structure altered from the native state of the raw starch. Raw starch preferably comprises corn, potato, rice, waxy maize, wheat, sago and/or tapioca starches which has not been chemically modified. The modified starch may be modified by enzyme, oxidation, crosslinking reaction, and/or substitution from raw starch. Preferably, the modified starches are chemically modified starches. The modified starch is nonionic starch, anionic starch or a mixture thereof. More preferably the modified starch is a combination of nonionic starch and anionic starch.

Nonionic starches mean modified starches that has been modified in a way such that they carry a neutral charge. Preferably, the nonionic starch is obtainable by esterification and/or etherification of raw starch. Preferably, the nonionic starch comprises hydroxyethylated starch, hydroxypropylated starch, acetylated starch, hydroxyethylated starch, hydroxypropylated starch, starch phosphate, starch sulfate or a combination thereof. More preferably, the nonionic starch comprises hydroxypropylated starch, hydroxyethylated starch, starch phosphate, starch sulfate or a combination thereof. Most preferably, the nonionic starch is hydroxypropyl starch phosphate. Anionic starches mean modified starches that have been modified in a way such that they carry a negative charge. Preferably, the anionic starch comprises starch modified by succinate and/or substituted succinate. More preferably the second modified starch comprises aluminium starch octenylsuccinate, sodium starch octenylsuccinate or a combination thereof.

Preferably the modified starch comprises hydroxyethylated starch, hydroxypropylated starch, acetylated starch, hydroxyethylated starch, hydroxypropylated starch, starch phosphate, starch sulfate, aluminium starch octenylsuccinate, sodium starch octenylsuccinate, calcium starch octenylsuccinate, sodium carboxymethyl starches, sodium starch glycolate or a combination thereof. More preferably the modified starch comprises hydroxyethylated starch, hydroxypropylated starch, acetylated starch, hydroxyethylated starch, hydroxypropylated starch, starch phosphate, starch sulfate, aluminium starch octenylsuccinate, sodium starch octenylsuccinate, calcium starch octenylsuccinate, sodium carboxymethyl starches, sodium starch glycolate or a combination thereof. Even more preferably the modified starch comprises hydroxypropylated starch, hydroxyethylated starch, starch phosphate, starch sulfate, aluminium starch octenylsuccinate, sodium starch octenylsuccinate or a combination thereof. Still even more preferably, the modified starch is selected from the group of hydroxypropyl starch phosphate, aluminium starch octenylsuccinate, sodium starch octenylsuccinate or a combination thereof. Most preferably, the modified starch is a combination of hydroxypropyl starch phosphate and aluminium starch octenylsuccinate.

The modified starch is present in amount of 0.01 to 10%, more preferably 0.2 to 5% and most preferably 0.5 to 3% by weight of the composition. Preferably, the weight ratio of nonionic starch to the anionic starch is 1:30 to 5:1, more preferably 1:15 to 2:1 and most preferably 1:10 to 1:1.

Preferably, the weight ratio of the total modified starches to the retinoid is 1:1 to 50:1, more preferably 2:1 to 22:1, and most preferably 3.5:1 to 12:1. In order to improve the stability of the composition, the weight ratio of the total modified starches to the sorbitan fatty acid ester is 1:1 to 70:1, more preferably 2:1 to 30:1, and most preferably 5:1 to 12:1.

The composition may comprise resorcinol derivative. Resorcinol derivative preferably refers to that at least one hydrogen on the ring structure and/or on a hydroxy group of the resorcinol replaced with an alkyl group, phenyl alkyl group. Preferably, the resorcinol derivative is 4-substituted resorcinol. Preferably, the resorcinol derivative is selected from 4-ethyl resorcinol, 4-butyl resorcinol, 4-hexyl resorcinol, phenylethyl resorcinol, or a mixture thereof, and more preferably, the resorcinol derivative comprises 4-hexyl resorcinol. The amount of the resorcinol derivative is preferably in the range of 0.00001 to 10%, more preferably from 0.001 to 5% and most preferably from 0.1 to 0.6% by weight of the total amount of the composition.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 to 10% by weight of the composition.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 96% by weight of the composition, more preferably from 25 to 92%, even more preferably from 42 to 88%, most preferably from 55 to 82% by weight of the composition.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

Preferably, the composition is an emulsion, more preferably an oil-in-water emulsion. The skin care composition refers to a composition suitable for topical application to human skin, including leave-on and wash-off products but preferably leave-on compositions. The term "leave-on" as used with reference to compositions herein means a composition that is applied to or rubbed on the skin and left thereon. The term "wash-off" as used with reference to compositions herein means a skin cleanser that is applied to or rubbed on the skin and rinsed off substantially immediately subsequent to application. Preferably the skin care composition means a fluid liquid, and particularly a moisturizer rather than a make-up product.

Preferably, the personal care composition is a skin care composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

The following examples are provided to facilitate an understanding of the invention. The examples are not intended to limit the scope of the claims.

### Examples

### Example 1

This example demonstrates effect of weight ratio of the sorbitan fatty acid ester to the retinoid on the color stability of personal care composition.

**Table 1**

| Ingredient | Samples (% by weight) | | | |
|---|---|---|---|---|
| | A | B | 1 | C |
| Water | To 100 | To 100 | To 100 | To 100 |
| Retinyl Propionate | 0.32 | 0.32 | 0.32 | 0.32 |
| Tween 20 ¹ | - | 0.1 | 0.25 | 0.5 |
| StarDesign^{™} Power ² | 2.00 | 2.00 | 2.00 | 2.00 |
| Xanthan Gum | 0.20 | 0.20 | 0.20 | 0.20 |
| Isopropyl Myristate | 10.00 | 10.00 | 10.00 | 10.00 |
| Hexylresorcinol | 0.40 | 0.40 | 0.40 | 0.40 |
| Niacinamide | 3.00 | 3.00 | 3.00 | 3.00 |
| Capric/Caprylic Triglyceride | 5.00 | 5.00 | 5.00 | 5.00 |
| Glycerin | 2.00 | 2.00 | 2.00 | 2.00 |
| 1,3-Butylene glycol | 3.00 | 3.00 | 3.00 | 3.00 |
| Cetyl Alcohol | 0.50 | 0.50 | 0.50 | 0.50 |
| Disodium EDTA | 0.05 | 0.05 | 0.05 | 0.05 |
| Tinogard^{®} TT | 0.10 | 0.10 | 0.10 | 0.10 |
| Tinogard^{®} DA | 0.10 | 0.10 | 0.10 | 0.10 |
| Preservative | 0.65 | 0.65 | 0.65 | 0.65 |

| | | | | |
|---|---|---|---|---|
| 1. Tween 20, supplied by CRODA 2. StarDesign^{™} Power, contains 10-40% of hydroxypropyl starch phosphate and 60-90% of sodium starch octenylsuccinate by weight, supplied by Cargill. | | | | |

A series of skin care compositions were formulated according to Table 1 by following standard procedures.

The performances of the samples were evaluated by the procedure as follows: all samples were put into same transparent bottles with identical amount. These transparent bottles with samples inside were placed into oven at 55°C for a week. Then, these transparent bottle with samples inside were taken out, observed, and recorded in Table 2.

**Table 2**

| Sample | Weight ratio of sorbitan fatty acid ester to retinoid | Score for discoloration |
|---|---|---|
| A | 0 | 2 |
| B | 0.31 | 1 |
| 1 | 0.78 | 0 |
| C | 1.56 | 3 |

| | | |
|---|---|---|
| *0: no discoloration; 1: slight discoloration; 2: noticeable discoloration; 3: obvious discoloration. | | |

It was surprisingly found that only when the weight ratio of the sorbitan fatty acid ester to the retinoid is within the range of the present invention, the discoloration problem of the composition was significantly reduced.

## Claims

1. A personal care composition comprising:
(a) retinoid;
(b) 0.01 to 10% of modified starch by weight of the composition; and
(c) sorbitan fatty acid ester,
wherein:
(i) the weight ratio of the sorbitan fatty acid ester to the retinoid is 0.4:1 to 1.4:1;
(ii) the weight ratio of the total modified starches to the sorbitan fatty acid ester is 1:1 to 70:1;
(iii) the sorbitan fatty acid ester is sorbitan C₈₋₂₄ fatty acid ester; and
(iv) the modified starch is nonionic starch, anionic starch or a mixture thereof.

2. The composition according to claim 1 wherein the retinoid comprises retinol, retinyl ester, or a mixture thereof, preferably the retinoid is selected from retinyl palmitate, retinyl propionate, or a mixture thereof.

3. The composition according to claim 1 or 2 wherein the retinoid is present in amount of 0.005% to 3%, preferably in an amount of 0.1% to 0.5% by weight of the composition.

4. The composition according to any one of the preceding claims wherein the sorbitan fatty acid ester is polyoxyethylene sorbitan fatty acid ester, the sorbitan fatty acid ester comprises Polysorbate 20, Polysorbate 40, Polysorbate 60, Polysorbate 80, or a mixture thereof.

5. The composition according to any one of the preceding claims wherein the sorbitan fatty acid ester is present in amount of 0.15 to 0.4% by weight of the composition.

6. The composition according to claim 4 or 5 wherein the weight ratio of the polyoxyethylene sorbitan fatty acid ester to the retinoid is 0.4:1 to 1.4:1, more preferably 0.55:1 to 1.2:1.

7. The composition according to any one of the preceding claims wherein the modified starch is selected from the group of hydroxypropyl starch phosphate, aluminium starch octenylsuccinate, sodium starch octenylsuccinate or a combination thereof.

8. The composition according to any one of the preceding claims wherein the modified starch is present in amount of 0.2 to 5% by weight of the composition.

9. The composition according to any one of the preceding claims wherein the weight ratio of the total modified starches to the retinoid is 1:1 to 50:1, preferably 3.5:1 to 12:1.

10. The composition according to any one of the preceding claims wherein the weight ratio of the total modified starches to the sorbitan fatty acid ester is 2:1 to 30:1, preferably 5:1 to 12:1.

11. The composition according to any one of the preceding claims wherein the composition comprise water in 42 to 88% by weight of the composition.

12. The composition according to any one of the preceding claims wherein the composition is an oil-in-water emulsion.

13. A non-therapeutic method for providing skin brightening, and/or anti-wrinkle benefit to skin comprising the step of applying a composition as claimed in any of the preceding claims 1 to 12 on the desired skin surface.

14. Non-therapeutic use of a composition of any one of claims 1 to 12 for providing skin brightening, and/or anti-wrinkle benefit on the desired skin surface.

## Patentansprüche

1. Körperpflegezusammensetzung, umfassend;:
(a) Retinoid;
(b) 0,01 bis 10% modifizierte Stärke, bezogen auf das Gewicht der Zusammensetzung; und
(c) Sorbitanfettsäureester,
wobei:
(i) das Gewichtsverhältnis des Sorbitanfettsäureesters zum Retinoid 0,4:1 bis 1,4:1 beträgt;
(ii) das Gewichtsverhältnis der gesamten modifizierten Stärken zum Sorbitanfettsäureester 1:1 bis 70:1 beträgt;
(iii) der Sorbitanfettsäureester ein Sorbitan-C₈₋₂₄-Fettsäureester ist; und
(iv) die modifizierte Stärke nichtionische Stärke, anionische Stärke oder eine Mischung davon ist.

2. Zusammensetzung nach Anspruch 1, wobei das Retinoid Retinol, Retinylester oder eine Mischung davon umfasst, wobei das Retinoid vorzugsweise unter Retinylpalmitat, Retinylpropionat oder einer Mischung davon ausgewählt ist.

3. Zusammensetzung nach Anspruch 1 oder 2, wobei das Retinoid in einer Menge von 0,005% bis 3%, vorzugsweise in einer Menge von 0,1% bis 0,5%, bezogen auf das Gewicht der Zusammensetzung, vorhanden ist.

4. Zusammensetzung nach irgendeinem der vorhergehenden Ansprüche, wobei der Sorbitanfettsäureester Polyoxyethylen-Sorbitanfettsäureester ist, wobei der Sorbitanfettsäureester Polysorbat 20, Polysorbat 40, Polysorbat 60, Polysorbat 80 oder eine Mischung davon ist.

5. Zusammensetzung nach irgendeinem der vorhergehenden Ansprüche, wobei der Sorbitanfettsäureester in einer Menge von 0,15 bis 0,4%, bezogen auf das Gewicht der Zusammensetzung, vorhanden ist.

6. Zusammensetzung nach Anspruch 4 oder 5, wobei das Gewichtsverhältnis des Polyoxyethylen-Sorbitanfettsäureesters zum Retinoid 0,4:1 bis 1,4:1, bevorzugter 0,55:1 bis 1,2:1 beträgt.

7. Zusammensetzung nach irgendeinem der vorhergehenden Ansprüche, wobei die modifizierte Stärke aus der Gruppe aus Hydroxypropylstärkephosphat, Aluminiumstärkeoctenylsuccinat, Natriumstärkeoctenylsuccinat oder einer Kombination davon ausgewählt ist.

8. Zusammensetzung nach irgendeinem der vorhergehenden Ansprüche, wobei die modifizierte Stärke in einer Menge von 0,2 bis 5%, bezogen auf das Gewicht der Zusammensetzung, vorhanden ist.

9. Zusammensetzung nach irgendeinem der vorhergehenden Ansprüche, wobei das Gewichtsverhältnis der gesamten modifizierten Stärken zum Retinoid 1:1 bis 50:1, vorzugsweise 3,5:1 bis 12:1, beträgt.

10. Zusammensetzung nach irgendeinem der vorhergehenden Ansprüche, wobei das Gewichtsverhältnis der gesamten modifizierten Stärken zum Sorbitanfettsäureester 2:1 bis 30:1, vorzugsweise 5:1 bis 12:1, beträgt.

11. Zusammensetzung nach irgendeinem der vorhergehenden Ansprüche, wobei die Zusammensetzung 42 bis 88% Wasser, bezogen auf das Gewicht der Zusammensetzung, umfasst.

12. Zusammensetzung nach irgendeinem der vorhergehenden Ansprüche, wobei die Zusammensetzung eine Öl-in-Wasser-Emulsion ist.

13. Nicht-therapeutisches Verfahren zum Aufhellen der Haut und/oder zur Vermeidung von Falten auf der Haut, umfassend den Schritt des Auftragens einer Zusammensetzung, wie in einem der vorhergehenden Ansprüche 1 bis 12 beansprucht, auf die die gewünschte Hautoberfläche.

14. Nicht-therapeutisches Verwendung einer Zusammensetzung nach irgendeinem der Ansprüche 1 bis 12 zum Aufhellen der Haut und/oder zur Vermeidung von Falten auf der gewünschten Hautoberfläche.

## Revendications

1. Composition de soins personnels comprenant:
(a) un rétinoïde;
(b) 0,01 à 10 % d'amidon modifié en poids de la composition; et
(c) un ester d'acide gras de sorbitane,
où:
(i) le rapport pondéral de l'ester d'acide gras de sorbitane au rétinoïde est de 0,4:1 à 1,4:1;
(ii) le rapport pondéral des amidons modifiés totaux à l'ester d'acide gras de sorbitane est de 1:1 à 70:1;
(iii) l'ester d'acide gras de sorbitane est un ester d'acide gras en C₈₋₂₄ de sorbitane; et
(iv) l'amidon modifié est un amidon non ionique, un amidon anionique ou un mélange de ceux-ci.

2. Composition selon la revendication 1, dans laquelle le rétinoïde comprend du rétinol, un ester de rétinyle ou un mélange de ceux-ci, de préférence le rétinoïde est choisi parmi le palmitate de rétinyle, le propionate de rétinyle ou un mélange de ceux-ci.

3. Composition selon la revendication 1 ou 2, dans laquelle le rétinoïde est présent en une quantité de 0,005 % à 3 %, de préférence en une quantité de 0,1 % à 0,5 % en poids de la composition.

4. Composition selon l'une quelconque des revendications précédentes, dans laquelle l'ester d'acide gras de sorbitane est un ester d'acide gras de polyoxyéthylène sorbitane, l'ester d'acide gras de sorbitane comprend du polysorbate 20, du polysorbate 40, du polysorbate 60, du polysorbate 80 ou un mélange de ceux-ci.

5. Composition selon l'une quelconque des revendications précédentes, dans laquelle l'ester d'acide gras de sorbitane est présent en une quantité de 0,15 à 0,4 % en poids de la composition.

6. Composition selon la revendication 4 ou 5, dans laquelle le rapport pondéral de l'ester d'acide gras de polyoxyéthylène sorbitane au rétinoïde est de 0,4:1 à 1,4:1, de préférence encore de 0,55:1 à 1,2:1.

7. Composition selon l'une quelconque des revendications précédentes, dans laquelle l'amidon modifié est choisi dans le groupe du phosphate d'hydroxypropylamidon, de l'octénylsuccinate d'amidon d'aluminium, de l'octénylsuccinate d'amidon de sodium ou une combinaison de ceux-ci.

8. Composition selon l'une quelconque des revendications précédentes, dans laquelle l'amidon modifié est présent en une quantité de 0,2 à 5 % en poids de la composition.

9. Composition selon l'une quelconque des revendications précédentes, dans laquelle le rapport pondéral des amidons modifiés totaux au rétinoïde est de 1:1 à 50:1, de préférence de 3,5:1 à 12:1.

10. Composition selon l'une quelconque des revendications précédentes, dans laquelle le rapport pondéral des amidons modifiés totaux à l'ester d'acide gras de sorbitane est de 2:1 à 30:1, de préférence de 5:1 à 12:1.

11. Composition selon l'une quelconque des revendications précédentes, dans laquelle la composition comprend de l'eau à raison de 42 à 88 % en poids de la composition.

12. Composition selon l'une quelconque des revendications précédentes, dans laquelle la composition est une émulsion huile-dans-eau.

13. Procédé non thérapeutique pour conférer un bénéfice éclaircissant la peau et/ou antirides à la peau, comprenant l'étape d'application d'une composition selon l'une quelconque des revendications 1 à 12 précédentes sur la surface de la peau souhaitée.

14. Utilisation non thérapeutique d'une composition selon l'une quelconque des revendications 1 à 12 pour conférer un bénéfice éclaircissant la peau et/ou antirides sur la surface de la peau souhaitée.
